(19) Europäisches Patentamt
European Patent Office
Office européen des brevets

(11) Veröffentlichungsnummer: **0 038 776**
**B1**

(12) # EUROPÄISCHE PATENTSCHRIFT

(45) Veröffentlichungstag der Patentschrift: **21.09.83**

(51) Int. Cl.³: **C 07 C 127/22**, A 01 N 47/34

(21) Anmeldenummer: **81810140.4**

(22) Anmeldetag: **13.04.81**

(54) **Phenylharnstoffe.**

(30) Priorität: **17.04.80 CH 2984/80**
**08.10.80 CH 7508/80**

(43) Veröffentlichungstag der Anmeldung: **28.10.81 Patentblatt 81/43**

(45) Bekanntmachung des Hinweises auf die Patenterteilung: **21.09.83 Patentblatt 83/38**

(84) Benannte Vertragsstaaten: **AT BE CH DE FR GB IT LI NL**

(56) Entgegenhaltungen: **US-A-3 933 908**

(73) Patentinhaber: **CIBA-GEIGY AG, Patentabteilung Postfach, CH-4002 Basel (CH)**

(72) Erfinder: **Ehrenfreund, Josef, Dr., Langenhagweg 11, CH-4123 Allschwil (CH)**
Erfinder: **Roloff, Achim, Dr., Waldshuterstrasse 9, CH-4310 Rheinfelden (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Phenylharnstoffe

Die vorliegende Erfindung betrifft neue substituierte N-Äthinylphenyl-N'benzolyharnstoffe, Verfahren zu ihrer Herstellung und ihre Verwendung in der Schädlingsbekämpfung.

Die erfindungsgemässen substituierten N-Äthinylphenyl-N'benzoylharnstoffe haben die Formel

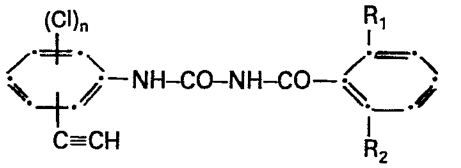

(I),

worin

$R_1$ Fluor, Chlor, Brom, Methoxy oder Methyl;

$R_2$ Wasserstoff, Fluor, Chlor, Brom, Methoxy oder Methyl; und n eine der Zahlen 0 oder 1 bedeuten, wobei der Äthinylrest -C≡CH sich in 3- oder 4-Stellung befindet.

Wegen ihrer Wirkung als Schädlingsbekämpfungsmittel bevorzugt sind solche Verbindungen der Formel I, die dadurch gekennzeichnet sind, dass sich der Äthinylrest -C≡CH in 4-Stellung befindet und solche, worin $R_1$ Fluor, Chlor, Brom oder Methyl, $R_2$ Wasserstoff, Fluor oder Chlor und n 0 bedeuten. Wertvoll sind aufgrund ihrer biologischen Wirksamkeit ferner die Verbindungen der Formel I, worin $R_1$ Fluor oder Chlor und $R_2$ Fluor bedeuten. Besonders wirksam sind erfindungsgemässe Verbindungen der Formel I, bei denen $R_1$ Chlor oder Methyl und $R_2$ Wasserstoff bedeuten.

Die Verbindungen der Formel I können nach an sich bekannten Verfahren hergestellt werden (vgl. u.a. die deutschen Offenlegungsschriften Nr. 2.123.236, 2.601.780 und die europäische Patentanmeldung Nr. 13414).

So kann man z.B. eine Verbindung der Formel I erhalten durch Umsetzung

a) einer Verbindung der Formel II

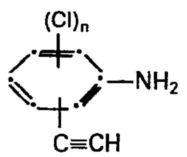

(II)

mit einer Verbindung der Formel III

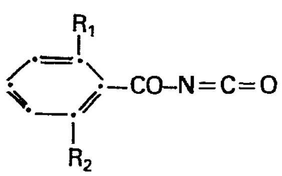

(III)

oder

b) einer Verbindung der Formel IV

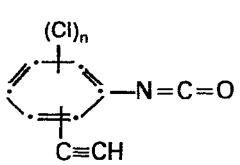

(IV)

mit einer Verbindung der Formel V

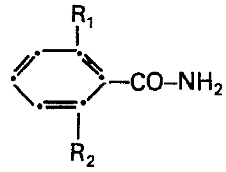

(V)

oder

c) einer Verbindung der Formel II mit einer Verbindung der Formel VI

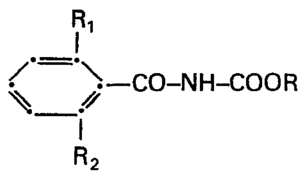

(VI)

In den obigen Formeln II, III, IV, V und VI haben die Reste $R_1$, $R_2$ sowie n die unter Formel I vorstehend angegebenen Bedeutungen und R bedeutet einen $C_1$-$C_8$-Alkylrest, der gegebenenfalls mit Halogen substituiert ist.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z.B. Äther und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von -10 bis 100 °C, vorzugsweise zwischen 15 und 25 °C, gegebenenfalls in Gegenwart einer organischen Base, z.B. Triäthylamin durchgeführt. Die Durchführung von Verfahren b) erfolgt bei einer Temperatur von 0 bis 120 °C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium. Für das Verfahren c), d.h. für die Umsetzung der Urethane der Formel VI mit den Aminophenylacetylenen der Formel II, werden Temperaturen zwischen etwa 60 °C und dem Siedepunkt des jeweiligen Reaktionsgemisches bevorzugt, wobei als Lösungsmittel insbesondere aromatische Kohlenwasserstoffe, wie Toluol, Xylol, Chlorbenzol usw., verwendet werden.

Die Ausgangsstoffe der vorstehenden Formeln II, III, IV, V und VI sind bekannt oder lassen sich, falls sie neu sind, analog bekannten Verfahren

herstellen. So kann man die Aminophenylacetylene der Formel II erhalten, indem man zunächst 1,1-Dimethyl-2-propin-1-ol in Gegenwart geeigneter Katalysatoren in alkalischem Medium mit den entsprechenden substituierten 2- bzw. 4-Halogennitrobenzolen bzw. anilinen (deren Aminogruppe geschützt ist) unter Halogenwasserstoffaustritt umsetzt (vgl. Tetrahedron Letters 1975, 4467; J. Organomet. Chem. 93/1973, 253 u. 259; italienische Patentschrift Nr. 1.006.879; US-Patentschrift Nr. 4.128.588). Die so erhaltenen Verbindungen der nachstehenden Formel VII werden dann alkalisch unter Abspaltung von Aceton zu Verbindungen der Formel VIII umgesetzt (vgl. J. Org. Chem. 44/1979, 1233; deutsche Offenlegungsschrift Nr. 2.905.507):

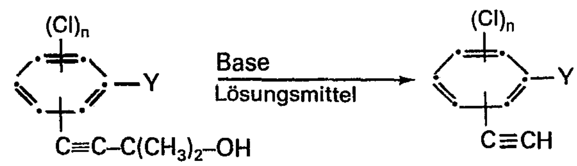


(VII) (VIII)

In den Formeln VII und VIII hat n die vorstehend angegebene Bedeutung, wobei sich der Äthinylrest in 3- oder 4-Stellung befindet und Y für eine Nitrogruppe oder eine geschützte Aminogruppe steht. Die Verbindung der Formel VIII kann dann nach allgemein üblichen Arbeitsweisen durch Reduktion der Nitrogruppe bzw. Abspaltung der Schutzgruppe in eine Verbindung der Formel II übergeführt werden.

3-Aminophenylacetylen und 4-Aminophenylacetylen gemäss Formel II sind bereits bekannte Verbindungen (vgl. J. Org. Chem. 44/1979, 1233 und 3671 bzw. Tetrahedron Letters 5/1959, 351).

Zu den Benzamiden der Formel V und den Benzoylisocyanaten der Formel III kann man unter anderem, ausgehend von den entsprechenden Nitrilen, wie folgt gelangen (vgl. J. Agr. Food Chem. 21, 348 und 993, 1973):

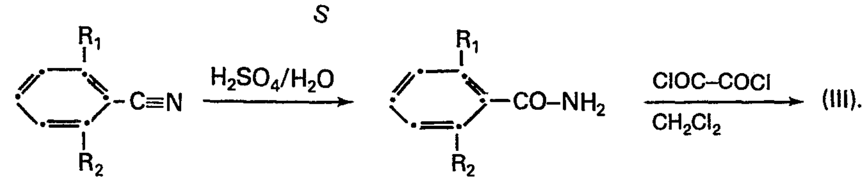


(III).

(V)

Die Phenylisocyanate der Formel IV lassen sich z.B. durch Phosgenisierung der entsprechenden Aminophenylacetylene der Formel II nach allgemein üblichen Verfahren herstellen. Die vorerwähnten als Ausgangsstoffe zu verwendenden Benzamide der Formel V sind zumeist bekannt (vgl. Beilstein «Handbuch der organischen Chemie» Bd. 9. S. 336). Die Urethane der Formel VI können in an sich bekannter Weise erhalten werden durch Umsetzung eines Benzoylisocyanats der Formel III mit einem entsprechenden Alkohol oder durch Umsetzung eines Benzamides der Formel V in Anwesenheit einer basischen Verbindung mit einem entsprechenden Ester der Chlorameisensäure.

Es ist bereits bekannt, dass bestimmte substituierte N-Phenyl-N'-benzoylharnstoffe pestizide bzw. insektizide Eigenschaften besitzen (vgl. deutsche Offenlegungsschriften Nr. 2.123.236, 2.531.279, 2.601.780, 2.726.684, 2.801.316 und 2.820.696, die europäischen Patentanmeldungen Nr. 1203 und 4030 sowie die US-Patentschrift Nr. 4.089.975). Aus J. Agr. Food Chem. 21, 348 ff. (1973) und 24, 134 (1976) sind weiterhin substituierte N-Phenyl-N'-2,6-dichlorbenzoylharnstoffe bekannt, die insektizide Eigenschaften aufweisen sollen.

Überraschenderweise wurde demgegenüber gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel besitzen. Sie eignen sich vor allem zur Bekämpfung von Pflanzen und Tiere befallenden Schädlingen.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Neben ihres sehr günstigen Wirkung gegenüber Fliegen, wie z.B. Musca domestica, und Mückenlarven eigenen sich Verbindungen der Formel I auch zur Bekämpfung von pflanzenschädigenden Frassinsekten, in Zier- und Nutzpflanzungen, insbesondere in Baumwollkulturen (z.B. gegen Spodoptera littoralis und Heliothis virescens) sowie in Obst- und Gemüsekulturen (z.B. gegen Laspeyresia pomonella, Leptinotarsa decemlineata und Pieris brassicae). Hervorzuheben ist besonders die hohe ovizide bzw. ovolarvizide Wirkung von Verbindungen der Formel I. Werden Verbindungen der Formel I von adulten Insekten mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I eignen sich weiterhin zur Bekämpfung von Ektoparasiten an Haus- und Nutztieren, z.B., durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z.B. folgende Wirkstoffe in Betracht:
organische Phosphorverbindungen,
Nitrophenole und Derivate,
Formamide, Harnstoffe, Pyrethroide,
Carbamate, chlorierte Kohlenwasserstoffe und Bacillus thuringiensis-Präparate.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithiote.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen, wie z.B. natürlichen oder regenerierten Stoffen, Lösungs-, Dispergier-, Nezt-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind «cattle dips», d.h. Viehbäder, und «spray races», d.h. Sprühgänge, in denen wässrige Zubereitungen verwendet werden, zu erwähnen. Diese Zubereitungsformen sind insbesondere zur Bekämpfung tierparasitärer Schädlinge geeignet.

Die Herstellung erfindungsgemässer Mittel erfolgt in an sich bekannter Weise durch inniges Vermischen und/oder Vermahlen von Wirkstoffen der Formel I mit den geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate);
Flüssige Aufarbeitungsformen:

a) in Wasser dispergierbare Wirkstoffkonzentrate: Spritzpulver (wettable powders), Pasten, Emulsionen;
b) Lösungen.

Der Gehalt an Wirkstoff in den oben beschriebenen Mitteln liegt zwischen 0,1 bis 95 Gew.-%.

Die Wirkstoffe der Formel I können beispielsweise wie folgt formuliert werden:

Stäubemittel: Zur Herstellung eines a) 5%igen und b) 2%igen Stäubemittels werden die folgenden Stoffe verwendet:
a) 5 Teile Wirkstoff,
95 Teile Talkum;
b) 2 Teile Wirkstoff,
1 Teil hochdisperse Kieselsäure,
87 Teile Talkum.

Die Wirkstoffe werden mit den Trägerstoffen vermischt und vermahlen.

Granulat: Zur Herstellung eines 5%igen Granulats werden die folgenden Stoffe verwendet:
5 Teile Wirkstoff,
0,25Teile epoxydiertes Pflanzenöl,
0,25Teile Cetylpolyglykoläther,
3,50Teile Polyäthylenglykol,
91 Teile Kaolin (Korngrösse 0,3–0,8 mm).

Die Aktivsubstanz wird mit dem epoxydierten Pflanzenöl in 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40%igen, b) und c) 25%igen, d) 10%igen Spritzpulvers werden folgende Bestandteile verwendet:
a) 40 Teile Wirkstoff,
5 Teile Ligninsulfonsäure-Natriumsalz,
1 Teil Dibutylnaphthalinsulfonsäure-Natriumsalz,
54 Teile Kieselsäure;
b) 25 Teile Wirkstoff,
4,5 Teile Calcium-Ligninsulfonat,
1,9 Teile Champagne-Kreide/Hydroxyäthylcellusose-Gemisch (1:1),
1,5 Teile Natrium-dibutyl-naphthalinsulfonat
19,5 Teile Kieselsäure
19,5 Teile Champagne-Kreide,
28,1 Teile Kaolin;
c) 25 Teile Wirkstoff,
2,5 Teile Isooctylphenoxy-polyoxyäthylen-äthanol,
1,7 Teile Champagne-Kreide/Hydroxyäthylcellulose-Gemisch (1:1),
8,3 Teile Natrimaluminiumsilikat,
16,5 Teile Kieselgur,
46 Teile Kaolin;
d) 10 Teile Wirkstoff,
3 Teile Gemisch der Natiumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate: Zur Herstellung eines 10%igen emulgierbaren Konzentrates werden folgende Stoffe verwendet:
10 Teile Wirkstoff
3,4 Teile epoxydiertes Pflanzenöl,

3,4 Teile eines Kombinationsemulgators, bestehend aus Fettalkoholpolyglykoläther und Alkylaralkyl-sulfonat-Calcium-Salz,
40 Teile Dimethylformamid,
43,2 Teile Xylol.

Aus einem solchen Konzentrat können durch Verdünnen mit Wasser Emulsionen jeder gewünschten Konzentration hergestellt werden.

Sprühmittel: Zur Herstellung eines 5%igen Sprühmittels werden die folgenden Bestandteile verwendet:
5 Teile Wirkstoff,
1 Teil epoxydiertes Pflanzenöl,
94 Teile Benzin (Siedegrenzen 160–190 °C);

Beispiel 1

2,0 g 4-Aminophenylacetylen werden in 20 ml absolutem Äther vorgelegt und unter Rühren werden 3,2 g 2,6-Difluorbenzoylisocyanat zugetropft. Der ausfallende Niederschlag wird nach 2 Stunden abgesaugt und aus Äthanol umkristallisiert. Man erhält N-(4-Äthinylphenyl)-N'-(2,6-difluorbenzoyl)-harnstoff vom Smp. 213–217 °C (Verbindung Nr. 1).

Analog den vorstehend beschriebenen Arbeitsweisen werden die folgenden Verbindungen der Formel I hergestellt:

| Verbindung Nr. | $R_1$ | $R_2$ | Stellung der Äthinylgruppe | $(Cl)_n$ | Schmelzpunkt [°C] |
|---|---|---|---|---|---|
| 1 | F | F | 4-C≡CH | n=0 | 213–217 |
| 2 | Cl | H | 4-C≡CH | n=0 | 189–192 |
| 3 | F | H | 4-C≡CH | n=0 | 213–214,5 |
| 4 | F | Cl | 4-C≡CH | n=0 | 158–161 |
| 5 | Cl | Cl | 4-C≡CH | n=0 | 215–217 |
| 6 | Br | H | 4-C≡CH | n=0 | 191–193 |
| 7 | $CH_3$ | H | 4-C≡CH | n=0 | 188–189,5 |
| 8 | $CH_3O$ | Cl | 4-C≡CH | n=0 | 184–185 |
| 9 | F | H | 4-C≡CH | 3-Cl | 220 |
| 10 | F | F | 4-C≡CH | 3-Cl | 220–223 |
| 11 | Cl | H | 4-C≡CH | 3-Cl | 202–205 |
| 12 | Cl | F | 4-C≡CH | 3-Cl | 199–201 |
| 13 | Cl | Cl | 4-C≡CH | 3-Cl | 208–212 |
| 14 | Cl | H | 3-C≡CH | 4-Cl | 205–207 |
| 15 | F | F | 3-C≡CH | 4-Cl | 242–244 |
| 16 | F | Cl | 3-C≡CH | 4-Cl | 210–213 |
| 17 | $CH_3$ | H | 3-C≡CH | 4-Cl | 222–223 |
| 18 | F | F | 3-C≡CH | n=0 | 173–176 |
| 19 | F | Cl | 3-C≡CH | n=0 | 225 |
| 20 | F | H | 3-C≡CH | n=0 | 165–168 |
| 21 | Cl | Cl | 3-C≡CH | n=0 | 219–221 |
| 22 | Cl | H | 3-C≡CH | n=0 | 185–188 |
| 23 | Br | H | 3-C≡CH | n=0 | 174–176 |
| 24 | $CH_3$ | H | 3-C≡CH | n=0 | 181–182 |
| 25 | F | Cl | 4-C≡CH | 2-Cl | |
| 26 | Cl | H | 4-C≡CH | 2-Cl | |
| 27 | Cl | Cl | 4-C≡CH | 2-Cl | |

Beispiel 2:
Wirkung gegen Musca domestica:

Je 50 g frisch zubereitetes CSMA-Nährsubstrat für Maden wurde in Becher eingewogen. Von einer 1 Gew.-%igen acetonischen Lösung des betreffenden Wirkstoffes wurde eine bestimmte Menge auf das in den Bechern befindliche Nährsubstrat pipettiert. Nach dem Durchmischen des Substrates lässt man das Aceton mindestens 20 Stunden lang verdampfen.

Dann wurden pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen wurden gezählt (toxischer Einfluss des Wirkstoffes auf die Madenentwicklung). Dann wurde nach 10 Tagen die Anzahl der aus den Puppen geschlüpften Fliegen bestimmt.

Verbindungen gemäss Beispiel 1 zeigten gute Wirkung im obigen Test.

Beispiel 3
Wirkung gegen Lucilia sericata:

Zu 9 ml eine Zuchtmediums wurde bei 50 °C 1 ml einer 0,5% Aktivsubstanz enthaltenden wässrigen Zubereitung gegeben. Nun wurden ca. 30 frisch geschlüpfte Lucilia sericata-Larven zum Zuchtmedium gegeben und nach 48 und 96 Stunden die insektizide Wirkung durch Ermittlung der Abtötungsrate festgestellt.

Verbindungen gemäss Beispiel 1 zeigten in diesem Test gute Wirkung gegen Lucilia sericata.

Beispiel 4

Wirkung gegen Aëdes aegypti:

Auf die Oberfläche von 150 ml Wasser, das sich in einem Behälter befindet, wurde so viel einer 0,1%igen acetonischen Lösung des Wirkstoffes pipettiert, dass Konzentrationen von je 10,5 und 1 ppm erhalten wurden. Nach Verdunsten des Acetons wurde der Behälter mit 30–40 3-tägigen Aedes-Larven beschickt. Nach 1, 2 und 5 Tagen wurde die Mortalität geprüft.

Verbindungen gemäss Beispiel 1 zeigten in diesem Test gute Wirkung gegen Aëdes aegypti.

Beispiel 5

Insektizide Frassgift-Wirkung:

Ca. 25 cm hohe eingetopfte Baumwollpflanzen wurden mit einem wässrigen Wirkstoffemulsionen besprüht, die den Wirkstoff in Konzentrationen von 800, 400, 200, 100 und 50 ppm enthielten.

Nach dem Antrocknen des Sprühbelages wurden die Baumwollpflanzen mit Spodoptera littoralis- bzw. Heliothis virescens-Larven im dritten larvalen Stadium besiedelt. Der Versuch wurde bei 24 °C und 60% relativer Luftfeuchtigkeit durchgeführt. Nach 120 Stunden wurde die %-Mortalität der Test-Insekten ausgewertet.

Beispiel 6

Wirkung auf Spodoptera littoralis und Heliothis virescens (Larven und Eier):

Es wurden drei in Töpfen gezogene Baumwollpflanzen von ca. 15–20 cm Höhe mit einer sprühfähigen flüssigen Zubereitung des zu prüfenden Wirkstoffes behandelt. Nach Antrocknen des Sprühbelages wurden die eingetopften Pflanzen in ein Blechgefäss von etwa 20 Litern Inhalt gestellt, das mit einer Glasplatte abgedeckt wurde. Die Feuchtigkeit im Inneren des abgedeckten Gefässes wurde so reguliert, dass sich kein Kondenswasser bildete. Direktes, auf die Pflanzen fallendes Licht wurde vermieden. Dann wurden die Pflanzen infiziert, und zwar insgesamt mit:

a) 50 Larven von Spodoptera littoralis oder Heliothis virescens des ersten larvalen Stadiums;

b) 20 Larven von Spodoptera littoralis oder Heliothis virescens des dritten larvalen Stadiums;

c) zwei Eispiegeln von Spodeptera littoralis oder Heliothis virescens. Dazu wurden je 2 Blätter einer Pflanze in einem beidseitig mit Gaze verschlossenen Plexiglaszylinder eingeschlossen; zwei Eispiegel von Spodoptera oder ein Teil eines Baumwollblattes mit darauf abgelegten Eiern von Heliothis wurden zu den eingeschlossenen Blättern gegeben.

Nach 4 bis 5 Tagen erfolgte die Auswertung gegenüber unbehandelten Kontrollen unter Berücksichtigung folgender Kriterien:

a) Anzahl der noch lebenden Larven,

b) Larvale Entwicklungs- und Häutungshemmung,

c) Frasschaden (Schabfrass und Lochfrass),

d) Schlupfrate (Anzahl der aus den Eiern geschlüpften Larven).

Die Verbindungen gemäss Beispiel 1 zeigten gute Gesamt-Wirksamkeit in obigem Test.

Beispiel 7

Ovizide Wirkung auf Spodoptera littoralis:

Auf Filterpapier abgelegte Eier von Spodoptera littoralis wurden aus dem Papier ausgeschnitten und in Lösungen des Wirkstoffes in einem Aceton-Wasser-Gemisch (1:1) getaucht. Die Behandlungs-Lösungen enthielten 400 bzw. 100 ppm des Wirkstoffs. Die so behandelten Eiablagen wurden dann aus diesem Gemisch herausgenommen und bei 21 °C und 60% relativer Feuchtigkeit in Kunststoffschalen deponiert.

Nach 3 bis 5 Tagen wurde die Schlupfrate, d.h. die Anzahl Larven, die sich aus den behandelten Eiern entwickelt hatten, bestimmt.

Beispiel 8

Ovizide Wirkung auf Heliothis virescens.

Entsprechende Mengenanteile einer benetzbaren pulverförmigen Formulierung, enthaltend 25 Gew.-% des zu prüfenden Wirkstoffes, wurden mit jeweils soviel Wasser vermischt, dass sich wässrige Emulsionen von ansteigender Wirkstoffkonzentration ergaben.

In diese wirkstoffhaltigen Emulsionen wurden eintägige Eigelege von Heliothis auf Cellophan während drei Minuten eingetaucht und dann auf Rundfiltern abgenutscht. Die so behandelten Gelege wurden in Petrischalen ausgelegt und in der Dunkelheit aufbewahrt. Nach 6 bis 8 Tagen wurde die Schlupfrate im Vergleich zu unbehandelten Kontrollen festgestellt. Zur Auswertung wurde die zur 100%-igen Abtötung der Eier erforderliche minimale Wirkstoffkonzentration bestimmt.

Verbindungen gemäss Beispiel 1 zeigten in diesem Test gute ovizide Wirkung gegen den geprüften Schädling.

Beispiel 9

Wirkung auf Laspeyresia pomonella (Eier):

Abgelegte Eier von Laspeyresia pomonella, die nicht älter als 24 Stunden waren, wurden auf Filterpapier für 1 Minute in acetonisch-wässrige Lösungen enthaltend 800, 50 bzw. 3 ppm des zu prüfenden Wirkstoffes eingetaucht. Nach dem Antrocknen der Lösung wurden die Eier in Petrischalen ausgelegt und bei einer Temperatur von 28 °C belassen. Nach 6 Tagen wurde der prozentuale Schlupf aus den behandelten Eiern gegenüber unbehandelten Kontrollen bewertet.

Biologische Ergebnisse:

Die nachstehende Tabelle zeigt Ergebnisse biologischer Prüfungen erfindungsgemässer Verbindungen auf der Basis obiger biologischer Beispiele. Die Auswertung der Versuche erfolgte anhand der erhaltenen %-Mortalität unter Verbindung des folgenden Bewertungs-Index:

A: 80 – 100% Mortalität bei einer Konzentration von 12,5 ppm der geprüften Verbindung.

B: 80 – 100% Mortalität bei einer Konzentration von 50 ppm der geprüften Verbindung

C: 80 – 100% Mortalität bei einer Konzentration von 100 ppm der geprüften Verbindung

D: 80 – 100% Mortalität bei einer Konzentration von 200 ppm der geprüften Verbindung

E: 80 – 100% Mortalität bei einer Konzentration von 400 ppm der geprüften Verbindung

F: weniger als 80% Mortalität bei einer Konzentration von 800 ppm der geprüften Verbindung

–: nicht geprüft.

| Verbindung Nr. | pestizide Wirksamkeit<br>Spodoptera Larven (Beispiel 5) | Heliothis Larven (Beispiel 5) | Laspeyresia Eier (Beispiel 9) | Spodoptera Eier (Beispiel 7) |
|---|---|---|---|---|
| 1 | B | C | A | C |
| 2 | B | B | B | – |
| 3 | C | F | F | E |
| 4 | B | B | A | E |
| 5 | C | E | F | E |
| 6 | B | D | A | – |
| 7 | B | F | A | E |
| 8 | C | E | – | – |
| 10 | E | E | – | – |
| 11 | E | E | – | – |
| 12 | F | F | – | – |
| 13 | D | F | – | – |
| 14 | E | E | – | – |
| 15 | E | E | – | – |
| 16 | E | E | – | – |
| 17 | E | E | – | – |
| 18 | B | D | – | – |
| 19 | E | F | – | – |
| 20 | B | C | F | E |
| 21 | D | F | – | – |
| 22 | D | F | – | – |
| 23 | E | E | – | – |
| 24 | C | F | F | E |

**Patentansprüche**

1. Verbindungen der Formel I

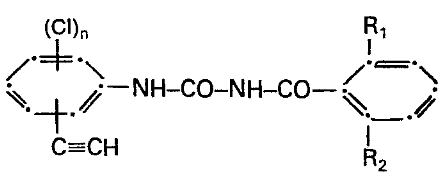

(I),

worin,

$R_1$ Fluor, Chlor, Brom, Methoxy oder Methyl;

$R_2$ Wasserstoff, Fluor, Chlor, Brom, Methoxy oder Methyl; und

n eine der Zahlen 0 oder 1 bedeuten, wobei der Äthinylrest -C≡CH sich in 3- oder 4-Stellung befindet.

2. Verbindung der Formel I gemäss Anspruch 1, dadurch gekennzeichnet, dass sich der Äthinylrest in 4-Stellung befindet.

3. Verbindungen der Formel I gemäss Anspruch 1, dadurch gekennzeichnet, dass

$R_1$ Fluor, Chlor, Brom oder Methyl;

$R_2$ Wasserstoff, Fluor oder Chlor und

n 0 bedeuten, wobei der Äthinylrest -C≡CH sich in 4-Stellung befindet.

4. Verbindungen der Formel I gemäss Anspruch 3, dadurch gekennzeichnet, dass

$R_1$ Fluor oder Chlor und

$R_2$ Fluor bedeuten.

5. Verbindungen der Formel I gemäss Anspruch 3, dadurch gekennzeichnet, dass

$R_1$ Chlor oder Methyl und

$R_2$ Wasserstoff bedeuten.

6. Verbindung gemäss Anspruch 4 der Formel

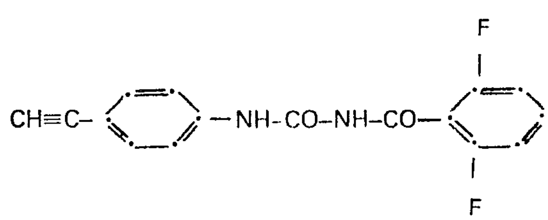

7. Verbindung gemäss Anspruch 5 der Formel

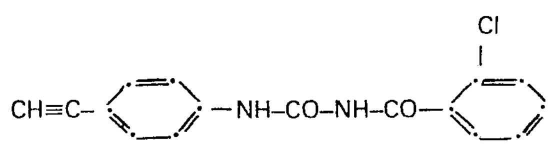

8. Verbindung gemäss Anspruch 4 der Formel

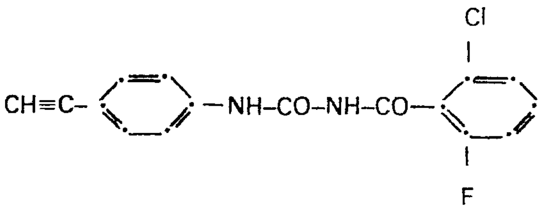

9. Verbindung gemäss Anspruch 5 der Formel

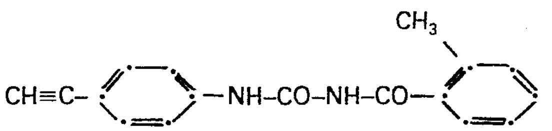

10. Verfahren zur Herstellung einer Verbindung gemäss den Ansprüchen 1 bis 9, dadurch gekennzeichnet, dass man

a) eine Verbindung der Formel II

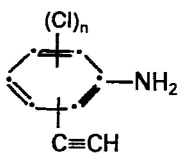 (II)

mit einer Verbindung der Formel III

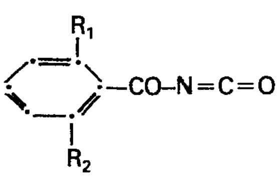 (III)

oder

b) eine Verbindung der Formel IV

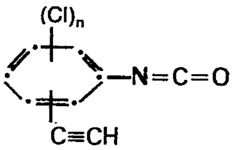 (IV)

mit einer Verbindung der Formel V

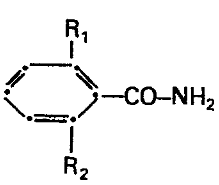 (V)

oder

c) eine Verbindung der Formel II mit einer Verbindung der Formel VI

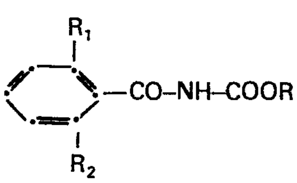 (VI)

umsetzt, wobei in den Formeln II bis VI die Reste $R_1$, $R_2$ sowie n die in den Ansprüchen 1 bis 5 angegebenen Bedeutungen haben und R für einen $C_1$–$C_8$-Alkylrest, der gegebenenfalls mit Halogen substituiert ist, steht.

11. Schädlingsbekämpfungsmittel, welches als aktive Komponente eine Verbindung gemäss den Ansprüchen 1 bis 9 zusammen mit geeigneten Trägern und/oder anderen Zuschlagstoffen enthält.

12. Verwendung von Verbindungen gemäss den Ansprüchen 1 bis 9 zur Bekämpfung von Schädlingen, vorzugsweise von Pflanzen und Tiere befallenden Insekten.

13. Verwendung gemäss Anspruch 12 als Ovizide zur Bekämpfung pflanzenschädigender Insekten.

**Revendications**

1. Composés de formule I

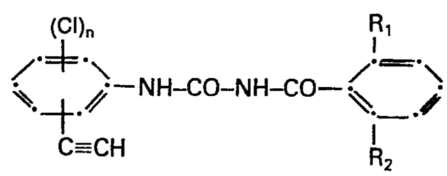 (I),

dans laquelle

$R_1$ représente le fluor, le chlore, le brome, un groupe méthoxy ou méthyle,

$R_2$ représente l'hydrogène, le fluor, le chlore, le brome, un groupe méthoxy ou méthyle; et

n est égal à 0 ou 1, le reste éthynyle -C≡CH se trouvant en position 3 ou 4.

2. Composé de formule I selon la revendication 1, caractérisé en ce que le reste éthynyle est en position 4.

3. Composés de formule I selon la revendication 1, caractérisés en ce que

$R_1$ représente le fluor, le chlore, le brome, un groupe méthyle,

$R_2$ représente l'hydrogène, le fluor ou le chlore, et n est égal à 0, le reste éthynyle -C≡CH se trouvant en position 4.

4. Composés de formule I selon la revendication 3, caractérisés en ce que

$R_1$ représente le fluor ou le chlore, et $R_2$ représente le fluor.

5. Composés de formule I selon la revendication 3, caractérisés en ce que $R_1$ représente le chlore ou un groupe méthyle, et $R_2$ représente l'hydrogène.

6. Composé selon la revendication 4, de formule

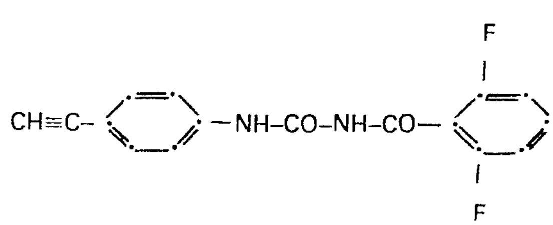

7. Composé selon la revendication 5, de formule

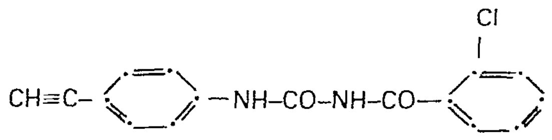

8. Composé selon la revendication 4, de formule

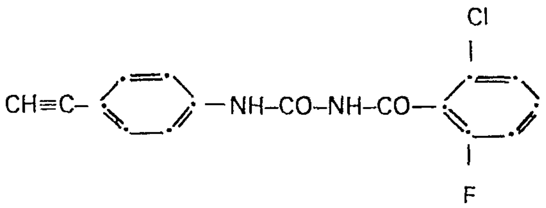

9. Composé selon la revendication 5, de formule

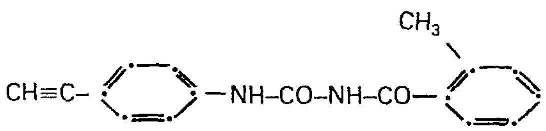

10. Procédé de préparation d'un composé selon les revendications 1 à 9, caractérisé en ce que:

a) on fait réagir un composé de formule II

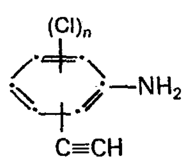

(II)

avec un composé de formule III

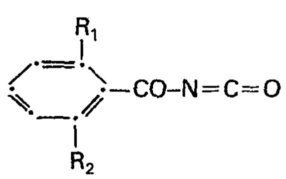

(III)

ou bien

b) on fait réagir un composé de formule IV

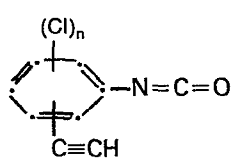

(IV)

avec un composé de formule V

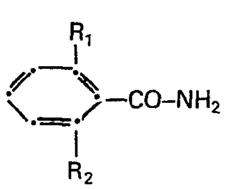

(V)

ou bien

c) on fait réagir un composé de formule II avec un composé de formule VI

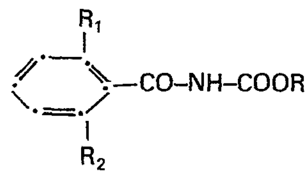

(VI)

les symboles $R_1$, $R_2$ et n ayant dans les formules II à VI les significations indiquées dans les revendications 1 à 5 et R représentant un groupe alkyle en $C_1$–$C_8$ éventuellement substitué par un halogène.

11. Produit pesticide contenant en tant que composant actif un composé selon les revendications 1 à 9 avec des véhicules et/ou d'autres additifs appropriés.

12. Utilisation des composés selon les revendications 1 à 9, dans la lutte contre les parasites, de préférence contre les insectes infestant les végétaux et les animaux.

13. Utilisation selon la revendication 12, en tant qu'ovicides dans la lutte contre les insectes nuisibles pour les végétaux.

**Claims**

1. A compound of the formula I

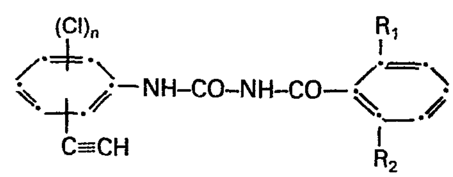

(I),

wherein $R_1$ is fluorine, chlorine, bromine, methoxy or methyl, $R_2$ is hydrogen, fluorine, chlorine, bromine, methoxy or methyl, and n is 0 or 1, and the ethynyl radical -C≡CH is in the 3 or 4 position.

2. A compound of the formula I according to claim 1, wherein the ethynyl radical is in the 4-position.

3. A compound of the formula I according to claim 1, wherein $R_1$ is fluorine, chlorine, bromine or methyl, $R_2$ is hydrogen, fluorine or chlorine, and n is 0, and the ethynyl radical -C≡CH is in the 4-position.

4. A compound of the formula I according to claim 3, wherein $R_1$ is fluorine or chlorine and $R_2$ is fluorine.

5. A compound of the formula I according to claim 3, wherein $R_1$ is chlorine or methyl and $R_2$ is hydrogen.

6. A compound according to claim 4 of the formula

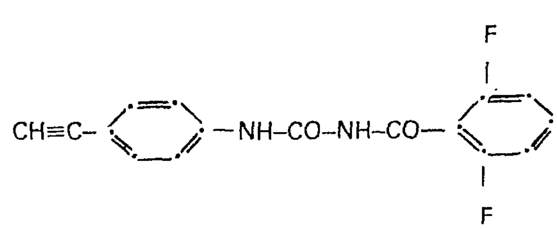

7. A compound according to claim 5 of the formula

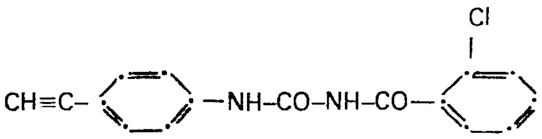

8. A compound according to claim 4 of the formula

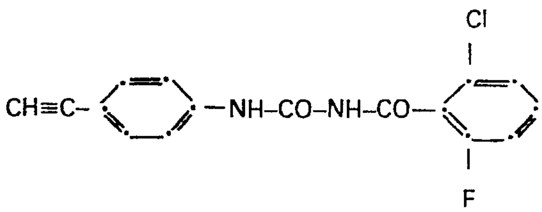

9. A compound according to claim 5 of the formula

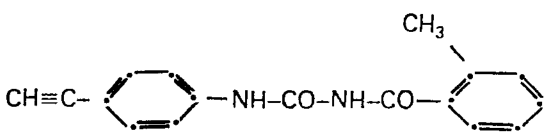

10. A process for the production of a compound according to any one of claims 1 to 9, which process comprises

a) reacting a compound of the formula II

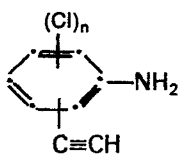 (II)

with a compound of the formula III

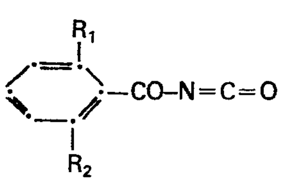 (III)

or

b) reacting a compound of the formula IV

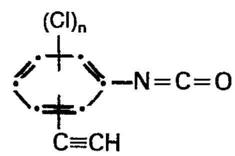 (IV)

with a compound of the formula V

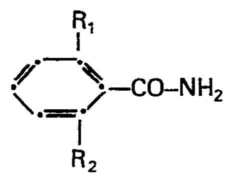 (V)

or

c) reacting a compound of the formula II with a compound of the formula VI

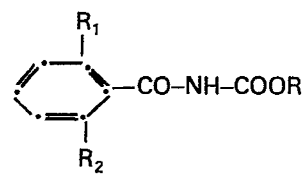 (VI)

in which formulae II to VI above, $R_1$, $R_2$ and n are as defined in claims 1 to 5 and R is a $C_1$–$C_8$alkyl radical which may be substituted by halogen.

11. A pesticidal composition which contains an effective amount of a compound according to any one of claims 1 to 9 together with suitable carries and/or other adjuvants.

12. A method of controlling pests at a locus, especially insects that attack plants and animals, which method comprises applying to said locus a pesticidally effective amount cf a compound according to any one of claims 1 to 9.

13. A method according to claim 12 for controlling plant-destructive insects, wherein the compound is applied as ovicide.